Europäisches Patentamt

European Patent Office

Office européen des brevets

(11) Publication number: **0 179 872 B1**

# EUROPEAN PATENT SPECIFICATION
## published in accordance with Art. 158(3) EPC

(45) Date of publication of patent specification: 19.06.91   (51) Int. Cl.⁵: **C12P 21/08**

(21) Application number: 85902341.8

(22) Date of filing: 22.04.85

(86) International application number:
**PCT/US85/00737**

(87) International publication number:
**WO 85/04811 (07.11.85 85/24)**

(54) **BISPECIFIC ANTIBODY DETERMINANTS.**

(30) Priority: **23.04.84 US 603125**

(43) Date of publication of application:
**07.05.86 Bulletin 86/19**

(45) Publication of the grant of the patent:
**19.06.91 Bulletin 91/25**

(84) Designated Contracting States:
**AT BE CH DE FR GB IT LI LU NL SE**

(56) References cited:
| | |
|---|---|
| EP-A- 0 068 763 | EP-A- 0 076 695 |
| EP-A- 0 096 463 | WO-A-83/03679 |
| US-A- 4 233 402 | US-A- 4 433 059 |
| US-A- 4 446 233 | US-A- 4 470 925 |
| US-A- 4 474 893 | US-A- 4 520 110 |

The Journal of Immunology, 125(6), issued 1980, Raso et al, 2610-2616

Journal of Experimental Medicine, 128, is-
sued 1968, Hämmerling et al, 1461-1469

Journal of Immunological Methods, 56, is-
sued 1983, Iamoyi et al, 235-245

(73) Proprietor: **BOSTON BIOMEDICAL RESEARCH
INSTITUTE, INC.
20 Staniford Street
Boston, MA 02114(US)**

(72) Inventor: **PAULUS, Henry, P.
85 East India Row
Boston, MA 02110(US)**

(74) Representative: **Moon, Donald Keith et al
BREWER & SON Quality House Quality Court
Chancery Lane
London WC2A 1HT(GB)**

## Description

BACKGROUND OF THE INVENTION

The IgG antibodies are known to consist of two half-molecules, each consisting of a light (L) chain and a heavy (H) chain. The H chains of the two halves are linked by disulfide bonds, which can be broken by selective reduction. If this step is performed for two different IgG samples, the half-molecules can be combined to form hybrid antibodies. This has been accomplished using intact rabbit globulins; Nisonoff et al. (1964) Science 134, 376-379. The IgA antibodies can also be split into identical half-molecules.

Hybrids have also been formed using the F-(ab')2 fragments of IgG antibodies, rather than intact antibodies; i.e., the F(c') portions of the molecules, which do not provide immunospecificity, are, prior to hybridization, removed by digestion with an appropriate protease such as pepsin. This procedure has been described in Nisonoff et al. (1960) Arch. Biochem. Biophys. 89, 230-244 and in Nisonoff and Rivers (1960) Arch. Biochem. Biophys. 93, 460-462. In a later discussion of the first paper Nisonoff wrote, in Current Contents (Nov. 2, 1981) 44, 25:
So far this procedure has had limited application, principally in the staining of cell surfaces with ferritin by using a hybrid of anti-ferritin antibody and antibody to a cell surface antigen. The use of hybrid antibody has also been considered as a means of bringing a pharmacological agent specifically into contact with a desired tissue surface.

The use of such hybrids for the delivery of cytotoxic drugs has also been suggested in Raso and Griffin (1978) Fed. Proc. 37, 1350.

Milstein (1981) Proc. R. Soc. Lond. B211, 393-412 suggests the possibility of using "monoclonal antibodies as carriers of toxic substances for specific treatment of tumors," and states that "(i)t is possible that Fab fragments will be better targeting agents than intact antibody."

Hybrid antibodies have also been formed by fusing two cells, each capable of producing different antibodies, to make a hybrid cell capable of producing hybrid antibodies. Such a method is described in Milstein & Cuello (1983) Nature 305, 537-540. Mouse hybridoma cells producing different monoclonal antibodies was fused and the resulting fused cells produced mixtures of antibodies composed of all possible combinations of the two different sets of parental heavy and light chains. Among this mixture of molecules were hybrid antibodies consisting of one half of each of the parental antibody molecules, which could be partially purified by ion exchange chromatography.

Another paper, Raso et al. (1981) Cancer Research 41, 2073-2078, describes the formation of an impure sample of rabbit antibody F(ab')2 fragments; the rabbit antibody fragments were split by reduction and reassembled with antiricin A chain F-(ab')2 fragments. The dual specificity dimers were used in targeted drug delivery experiments. The article states:
The two types of purified antibodies used for this work were isolated from conventional heteroantisera. Thus, a complicated array of affinity and specificity combinations must arise upon annealing these two populations. The advent of homogenous hybridoma-derived antibodies will afford absolute control over the binding affinities of the constituent halves of a hybrid antibody, and this uniformity should greatly boost their ultimate effectiveness as delivery vehicles.

The present invention provides a method of preparing a bispecific monoclonal antibody determinant comprising the steps of providing two different monoclonal antibody determinants, each said determinant comprising two identical L-H half-molecules linked by one or more disulfide bond,
subjecting said two different antibody determinants to conditions sufficient to break said disulfide bonds linking said L-H half-molecules, and under conditions which prevent the formation of disulfide bonds within the H-chains of said half-molecules, whereby each said determinant is split into a pair of identical half-molecules,
derivatizing one said pair of identical half-molecules with thiol activating agent, and
combining said half-molecules under conditions which permit said half-molecules to form said bispecific antibody determinants by the formation of one or more disulfide bonds.

Preferably the process further includes, prior to the combining step, derivatizing one pair of identical half-molecules with a thiol activating agent which facilitates the formation of the disulfide bonds between the thiol activated half-molecules and the different half-molecules.

In other preferred embodiments, the monoclonal antibodies are IgG (most preferably IgG , or, less preferably, IgG2A, IgG2B, or IgG3) or IgA; each half-molecule, if derived from an IgG antibody, includes at least the F(ab') portion; the thiol activating agent prevents the recombination of the thiol activated half-molecules; and the thiol activating agent is 5,5'-dithiobis (2-nitrobenzoic acid), 2,2'-dipyridine disulfide, 4,4-dipyridine disulfide, or a mixture of sulfite and tetrathionate. (The use of thiol activating agents to link F(ab') fragments to other proteins has been described, e.g., in Raso et al. (1980) J. Immunol. 125, 2610; Raso et al. (1982) Cancer Res. 42, 457; and Masuho et al. (1979) B.B.R.C. 90, 320, hereby incorporated by reference.) Preferably the formation of disulfide bonds within the H chains of half-molecules originally linked by disulfide bonds is prevented by carrying out the splitting reaction in the presence of a dithiol complexing agent such as an arsenite, e.g., an inorganic arsenite such as sodium arsenite, or an aryl arsenite, e.g., phenylarsine oxide, or a cadmium salt, or by carrying out the splitting reaction under conditions under which the conformation of the H chains is modified to prevent disulfide bond formation, e.g., at a pH between 3.8 and 4.5 (most preferably 4.2), or by removing all but one reduced cysteine residue using a proteolytic enzyme such as carboxypeptidase Y.

Where the recombination of like half-molecules with each other is prevented, purification of bispecific determinants from the mixture can be carried out simply on the basis of molecular size, e.g. by gel filtration; this is possible because the only molecules in the solution, half-molecules and bispecific hybrids, are different in size by a factor of two. Other separation methods are, e.g., ion exchange and isoelectric focusing.

Figure 1 is a diagrammatic representation of an assembly useful in a bioluminescence assay.

Figure 2 is a diagrammatic representation of a channeling immunoassay employing bispecific monoclonal antibody determinants.

Figure 3 is a diagrammatic representation of a fluorescent energy transfer immunoassay employing bispecific monoclonal antibody determinants.

Figure 4 is a diagrammatic representation showing the structure of a rabbit $IgG_1$ compared to mouse $IgG_1$.

Figure 5 is a diagrammatic representation of competing intrachain disulfide reactions in mouse half-molecules.

Figure 6 is a diagrammatic representation of a preferred method of preparing bispecific monoclonal antibody determinants.

Figure 7 is a diagrammatic representation of a luminescent energy transfer immunoassay employing bispecific monoclonal antibody determinants.

The bispecific monoclonal antibody determinants of the invention are useful for a wide range of applications. These applications all flow from the ability of these determinants to serve as highly specific linkers through specific sites B', of any two antigenic determinants capable of stimulating antibody production in animals; e.g., effective proteins, polypeptides, carbohydrates, nucleic acids, or haptens, either free or immobilized on surfaces or particles.

One application of the bispecific antibody determinants of the invention is their use as agents for bonding a desired antigenic entity to a desired surface which contains or has immobilized on it a different antigenic determinant. For example, enzymes so immobilized on particles or membranes can be used as solid-state catalysts. Advantages of this type of immobilization over others are that antibodies can be selected which have no adverse effect on enzyme activity, and that pure enzymes can be immobilized from impure mixtures. Bispecific antibody determinants can also be used as highly specific bispecific reagents for immunoassay procedures which are used, e.g., in the diagnosis of medical disorders, or as molecular probes to study the relationships between antigenic determinants in biological systems.

An additional application of the bispecific antibody determinants is their use in electrodes. The invention can be used in electrodes of any configuration, e.g. in solid state sensors such as field effect transistors e.g., as described in Wohltjen (1984) Analyt. Chem. 56, 87A.

Enzyme electrodes made using bispecific antibody determinants possess several advantages over conventional enzyme electrodes. One advantage is their precise self-assembling property: the desired electrode assembly is generated simply by attaching the appropriate hapten or haptens to the membrane (either the electrode membrane or a separate membrane associated with the electrode) and then immersing the hapten-derived membrane into a solution containing the appropriate bispecific antibodies and enzymes. This ease of assembly also means that the electrode can be easily recharged after deterioration has occurred through prolonged use.

Another advantage of the electrodes is also a function of the specificity of the bispecific antibody determinants. Any given enzyme will possess a number of antigenic sites capable of binding to a specific site of an antibody. However, coupling at many of these sites can cause inactivation of the enzyme. In the case of bispecific monoclonal antibody determinants, this problem is avoided because the determinants are selected so that they couple with the enzyme only at a site which does not cause deactivation of the enzyme.

A further advantage is that assembly or recharging of the electrode can be done with impure enzyme mixtures because the unique specificity of the bispecific antibody determinants assures the selection of the proper enzymes from the impure mixture. In some instances, the electrode assembly can be stabilized with a mild bifunctional cross linker, e.g., dimethyl suberimidate.

Yet another application for the bispecific antibody determinants is their use in the formation of self-assembling networks for use, e.g., as molecular microciruits.

The above applications are described in more detail in Paulus U.S. Pat. No. 4,444,878, hereby incorporated by reference.

We turn now to a more detailed discussion of the formation of the bispecific antibody determinants of the invention, and to the particular method used where the antibodies are of mouse origin.

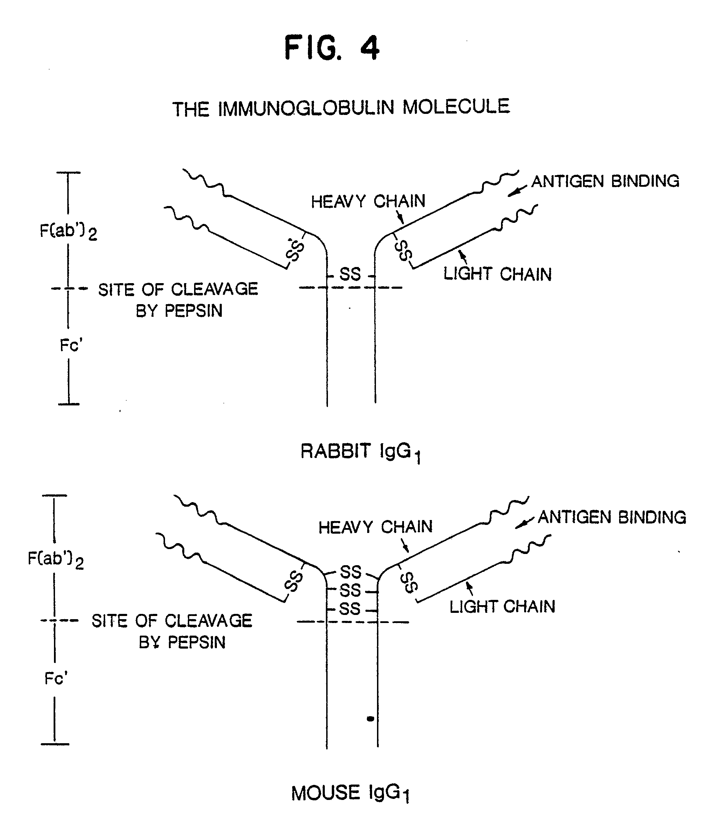

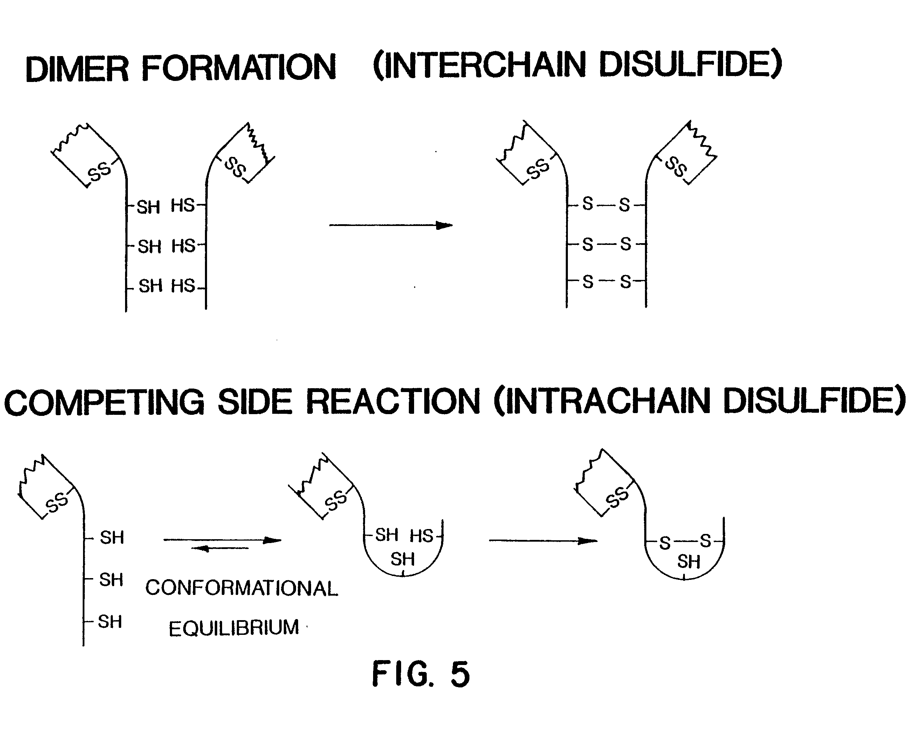

Figure 4 illustrates the difference in structure between rabbit IgG$_1$ antibodies and mouse IgG$_1$ antibodies (which comprise most monoclonal antibodies.) These structures differ in the number of disulfide bonds linking the heavy chains. The two halves of rabbit IgG$_1$ are held together by a single bond, and the reduction of F(ab')$_2$ to F(ab') monomer as well as the reassociation of monomer to the dimer thus involves the breakage and reformation of only one bond, a relatively simple process. In contrast, the heavy chains of mouse IgG$_1$ are linked by 3 disulfide bonds, and their breakage and reformation can lead to competing intrachain side reactions of the type illustrated in Figure 5, which can significantly diminish the yield of desired product. The intrachain reaction renders the half-molecule unavailable for combination with a different half-molecule. The method of the invention allows the production of pure bispecific monoclonal antibody determinants in high yield, from mouse monoclonal antibodies as well as from antibodies derived from other mammalian species.

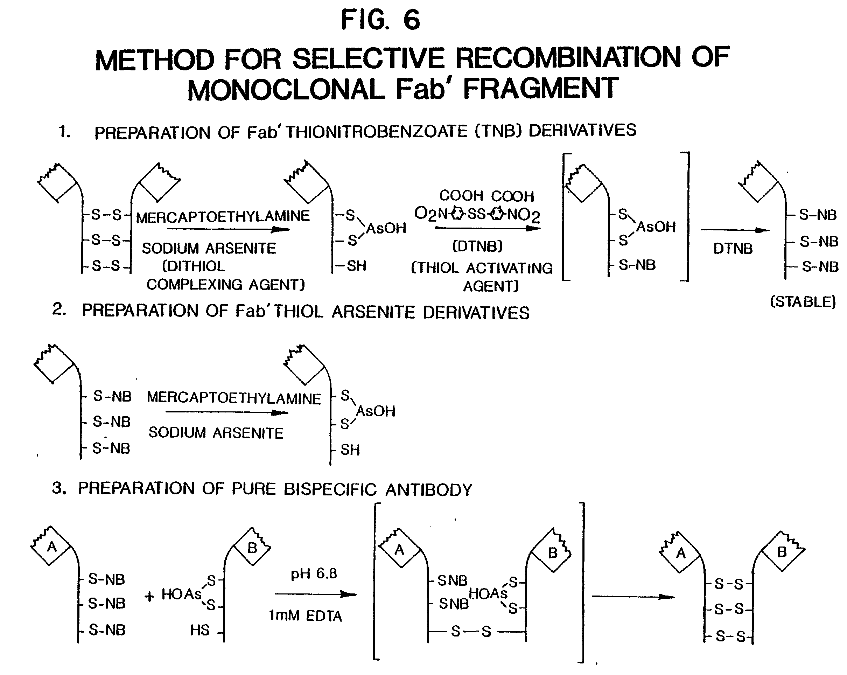

The method involves the following sequence of steps. Using conventional methods, two different monoclonal IgG$_1$ antibody samples are produced, each antibody having one of two desired specificities. Each sample is then exposed to an appropriate protease such as pepsin to cleave off the F(c') portion of the antibody molecule to produce an F(ab')$_2$ fragment. Each sample is then subjected to conditions sufficient to break the disulfide bonds linking the F(ab') half-molecules, but not any of the other disulfide bonds in the molecule. At the same time, these conditions are such as to prevent the formation of disulfides within a single heavy chain, for example by the addition of a dithiol complexing agent (e.g. sodium arsenite (as shown in Figure 6), an aromatic arsenite such as phenylarsine oxide, or CdCl$_2$), or by modifying the conformation of the heavy chain (e.g. by lowering the pH to 4.2), or by removing all but one of the reduced cysteine residues with a proteolytic enzyme (e.g. carboxypeptidase Y).

One of the samples is then exposed to a thiol-activating agent to complex all free thiols, forming a derivative that can react rapidly with other free thiols to form disulfides, as shown at the end of sequence 1 in Figure 6. Among the agents suitable for this purpose are aromatic disulfides such as DTNB (Ellman (1959) Arch. Biochem. Biophys. 82, 70) (Figure 6), 2,2'-dipyridine disulfide, 4,4'-dipyridine disulfide (Grasetti et al. (1967) Arch. Biochem. Biophys. 119, 41) and sulfite/tetrathionate (Masuho et al. (1979) B.B.R.C. 98, 320). This activated sample is then combined (sequence 3 of Figure 6) with the other dithiol complexed sample, which has available thiols, under conditions where at least some half-molecules combine chemically to form bispecific antibody determinants but no monospecific F(ab')$_2$.

Alternatively, both samples can be exposed to the thiol-activating agent to form the activated thiol derivatives. (This is frequently convenient because of the relatively good stability of the activated derivatives.) One of the samples is then treated with an excess of a low molecular weight thiol to regenerate the free thiols on the F(ab') monomer, followed by separation from excess low molecular thiols. Intramolecular disulfide bond formation can be avoided by the addition of a dithiol complexing agent (sequence 2 of Figure 6) or by modifying the conformation of the heavy chain. It may in some instances, however, be desirable to permit the formation of intramolecular disulfide bonds. This sample is then combined (sequence 3 of Figure 6) with the other F(ab') sample in the thiol-activated form under conditions where at least some half-molecules combine chemically to form bispecific F(ab')$_2$.

Since the F(ab')$_2$ fraction produced under those conditions consists only of the desired bispecific antibody determinants, provided appropriately purified monoclonal antibodies have been used as starting materials, homogenous bispecific antibody determinants can be obtained simply by removing the monomeric F(ab') fraction from the desired F(ab')$_2$ on the basis of molecular size, by a convenient procedure such as gel filtration.

The following procedure is used to prepare a homogenous sample of identical bispecific antibody determinants in which each bispecific determinant has a site specific for a unique antigenic site on the protein avidin, and a site specific for a unique antigenic site on the enzyme B-galactosidase. This procedure follows the steps generally illustrated in Figure 6.

The first step is the preparation of monoclonal antibodies against the two proteins avidin and B-galactosidase. This is done by first immunizing one group of BALB/C mice against each enzyme using standard immunization procedures.

Following immunization, spleen cells of immunized animals are prepared and fused with a derivative of MOPC-21 myeloma cells (e.g., NS1 or SP2/O-Ag14) using the procedure described in Galfre et al. (1981) Methods in Enzymology 73, 3-46. The hybrid cells are selected in hypoxanthine-aminopterin-thymidine medium, cloned, and screened for production of antibodies against the desired enzymes by the method described in Galfre et al. Id. The clones found to produce antibodies against the desired enzyme are then screened to select a clone which produces an antibody of the IgG₁ class which has a high affinity for the enzyme and which does not cause inactivation of the enzyme. The clones of interest are stored until use under liquid nitrogen. Antibody is prepared by the standard technique of propagating the cloned cells as ascitic tumors in the peritoneal cavities of pristane-primed mice.

The desired IgG₁ antibodies against avidin and B-galactosidase are then purified from ascites fluid essentially as described by Parham et al. (1982) Journal of Immunological Methods 53, 133-173, by a procedure involving ammonium sulfate precipitation, gel filtration, and chromatography on DEAE cellulose with a linear salt gradient. The monoclonal antibodies are cleaved to $F(ab')_2$ fragments with pepsin as described by Lamoyi and Nisonoff (1983) Journal of Immunological Methods 56, 235-243 by incubating for 18 hours at 25°C with pepsin (2% by weight) in 0.1 M sodium acetate, pH 4.2. The $F(ab')_2$ fragments are then purified by high performance liquid chromatography on a TSK 3000 SW column in 0.1 M sodium phosphate, pH 6.8, as described in Parham et al. Id.

The $F(ab')_2$ fractions are then completely converted to Fab' monomer by reduction with 1 mM 2-mercaptoethylamine in 1 mM EDTA, 10 mM sodium arsenite, and 0.1 M sodium phosphate, pH 6.8, for 18 hours at 25°C. The mixture is then supplemented with solid DTNB to final concentration of 20 mM. After 2-3 hours at 25°C, excess DTNB and low molecular weight products are removed by centrifugal gel filtration on Sephadex G-25 equilibrated with 0.1 M sodium phosphate, pH 6.8, and 1 mM EDTA. The resulting thionitrobenzoate derivatives of the Fab' monomers are relatively stable and can be stored for several days without significant decomposition.

One of the thionitrobenzoate derivates of Fab' monomer (derived from the anti-avidin antibody) is then converted back to the free thiol form (complexed with dithiol) by incubating for 30 minutes at 25°C with 10 mM mercaptoethylamine in 1 mM EDTA, and 0.1 M sodium phosphate, pH 6.8. The excess mercaptoethylamine and low molecular weight reaction products are then removed by centrifugal gel filtration on Sephadex G-25 equilibrated with 0.1 M sodium phosphate, pH 6.8, and 1 mM EDTA.

The resulting anti-avidin F(ab')-thiol is then contacted immediately with an equimolar amount of the thionitrobenzoate derivative of the anti-B-galactosidase Fab', at a protein concentration of 0.5 mg/ml or higher, for 3-20 hours at 25°C, to allow formation of bispecific $F(ab')_2$. The mixture is then supplemented with solid DTNB to a final concentration of 5 mM and left for 3 hours at 25°C to promote dissipation of any non-covalent dimeric material. Homogenous bispecific antibody determinant against avidin and B-galactosidase is then prepared by separating the $F(ab')_2$ fraction from residual Fab' monomers by high performance liquid chromatography on TSK 3000 SW in 0.1 M in sodium phosphate, pH 6.8, a procedure which does no damage to the $F(ab')_2$, and does not require its being bonded to anything else, minimizing the risk for conformational changes which could affect activity. The formation of the desired bispecific antibody determinant can be conveniently demonstrated by its ability to cause the avidin-dependent binding of B-galactosidase to disks of biotin-substituted cellulose.

The bispecific antibody determinant can be coupled to a biotin-substituted cellulose membrane in an assay for a compound, e.g., lactose, which B-galactosidase can help measure (as is explained in more detail below).

Anti-B-galactosidase can be immobilized on the biotin substituted membrane by simply contacting the membrane (which has avidin bonded to the biotin) with the bispecific determinant having an anti-avidin half and an anti-B-galactosidase half, and with B-galactosidase. In a similar manner, glucose oxidase can be immobilized to the membrane.

Using the same procedure described above, two bispecific molecules can be made, the first having specificity for a unique antigenic site on the enzyme glucose oxidase and for an antigenic site on B-galactosidase, and the second having specificity for a different antigenic site on glucose oxidase and for an antigenic site on Type I collagen. These can be used to form an electrode for measuring lactose generally, as described in Paulus, id.

The following is a description of an example of the type of assay which employs the measurement of a substance which can be measured colorimetrically, reflectometrically, luminescently, or fluorometrically, as a measure of an unknown amount of a substance being assayed. Generally, the assay measures the amount of an unknown substance in a liquid sample, which substance is acted on by at least a first enzyme to evolve a measurable ion or compound which can be used as a measure of the unknown substance. The assay involves linking the first enzyme to a solid support (e.g. a bead or a membrane support), or to a previously sequentially acting second enzyme, by means of a bispecific antibody determinant of the invention having specificity for the first enzyme and for the support or the second enzyme. As many sequential enzymes as are involved in the reaction sequence can be linked this way, with the last to act generally being linked to the support. The sample is contacted with the linked enzyme or enzymes immobilized on the support, and the measurable ion or compound is measured. Unknown substances which can be measured include, e.g., blood sugar.

Alternatively, the sequential enzymes can be linked indirectly through an unknown substance to be measured through appropriate bispecific antibodies. This extends the range of substances that can be measured to biomolecules such as hormones (e.g., human chorionic gonadotropin, measured in pregnancy tests, and insulin).

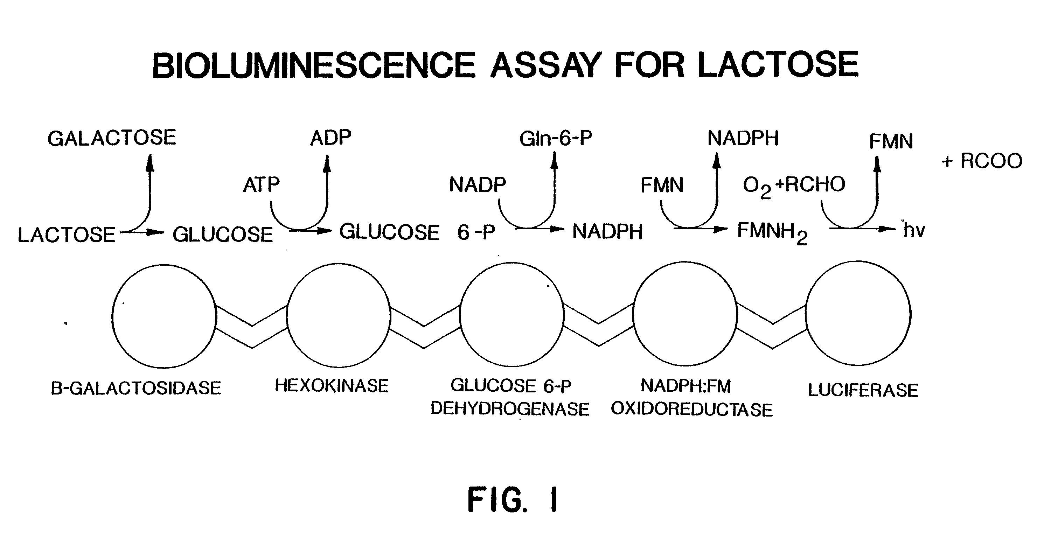

Figure 1 illustrates an arrangement of self-assembling enzymes linked by bispecific antibody determinants (shown as chevrons linking circular enzymes). The last enzyme in the sequence (luciferase) can be immobilized using, e.g. avidin and a biotin-substituted membrane (not shown). Luciferase, when reduced as shown, is bioluminescent (the reaction chain shown is described in greater detail in Wienhausen and DeLuca (1982) Anal. Biochem. 127, 380, and Hastings et al. U.S. Patent No. 4,278,761, hereby incorporated by reference).

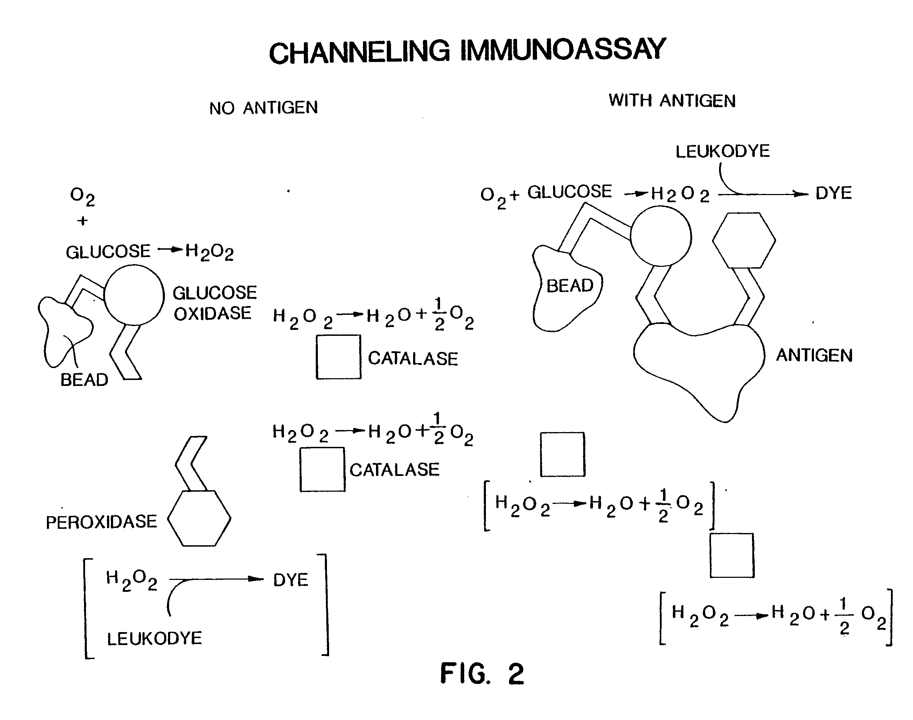

Figure 2 illustrates a "channeling" immunoassay which employs two bispecific monoclonal antibody determinants. (The illustrated assay employs some of the principles of the channeling immunoassay described in Litman et al. (1980) Anal. Biochem. 106, 223). The first bispecific determinant has specificity for a first enzyme and for a first antigenic site on an antigen being measured, e.g. human chorionic gonadotropin. The second bispecific determinant has specificity for a second enzyme and for a second antigenic site on the antigen being measured. The first enzyme is capable of acting on a first substrate to produce a second substrate which can be acted on by the second enzyme to evolve a measurable compound or ion. The assay is carried out by contacting the liquid mixture suspected of containing the molecule to be measured with the two bispecific determinants and the two enzymes, in the presence of the first substrate. If the unknown is present, the two determinants will bind to it and the two enzymes bound to them will be brought very close together, so that the efficiency of the two reactions is increased by orders of magnitude. In the illustrated scheme, the first enzyme is glucose oxidase, the second enzyme is peroxidase, the first substrate is glucose, and the second substrate is peroxide, which oxidizes a leukodye to produce a measurable dye. The last enzyme in the sequence can be immobilized. The assay illustrated in Fig. 2 includes an optical feature for improving the signal to noise ratio, the use of a competing enzyme (in this case, catalase) in an amount large enough to swamp the action of peroxidase in the absence of the enzyme-coupling unknown substance.

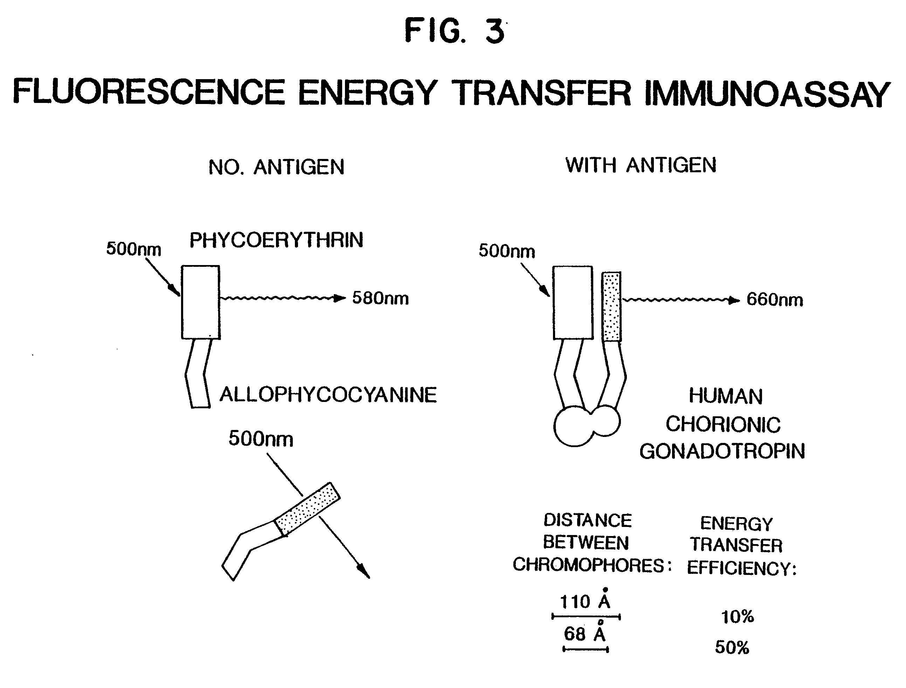

Figure 3 illustrates a fluorescent energy transfer immunoassay analogous to the assay of Figure 2; the difference is that in the energy transfer assay, light, rather than organic substrates, is sequentially acted on to produce a measurable result. The assay employs two fluorescent proteins, in the illustrated assay, these are phycoerythrin ("Phy") and allophycocyanine ("All"), both produced by blue-green algae and described, as being useful in immunoassays, in Glazer and Stryer (1983) Biophys. J. 43, 323, hereby incorporated by reference. As illustrated in Figure 3, one bispecific monoclonal antibody determinant has specificity for a first site on the antigen being assayed (e.g. human chorionic gonadotropin) and for Phy, and the second bispecific monoclonal antibody determinant has specificity for a second site on the antigen for All. Presence of the antigen closely couples Phy and All, greatly increasing energy transfer efficiency. The signal is produced because Phy excites at 500 nm and transmits at 580 nm, while All is transparent to 500 nm but excites at 580 nm and transmits at 660 nm. Thus the 660 nm signal is only achieved by the sequential passage of light through both proteins, a process whose efficiency depends on proximity of the proteins. As in the assays described above, one or both fluorescent proteins can be immobilized on a support, either by conventional means or via bispecific determinants of the invention.

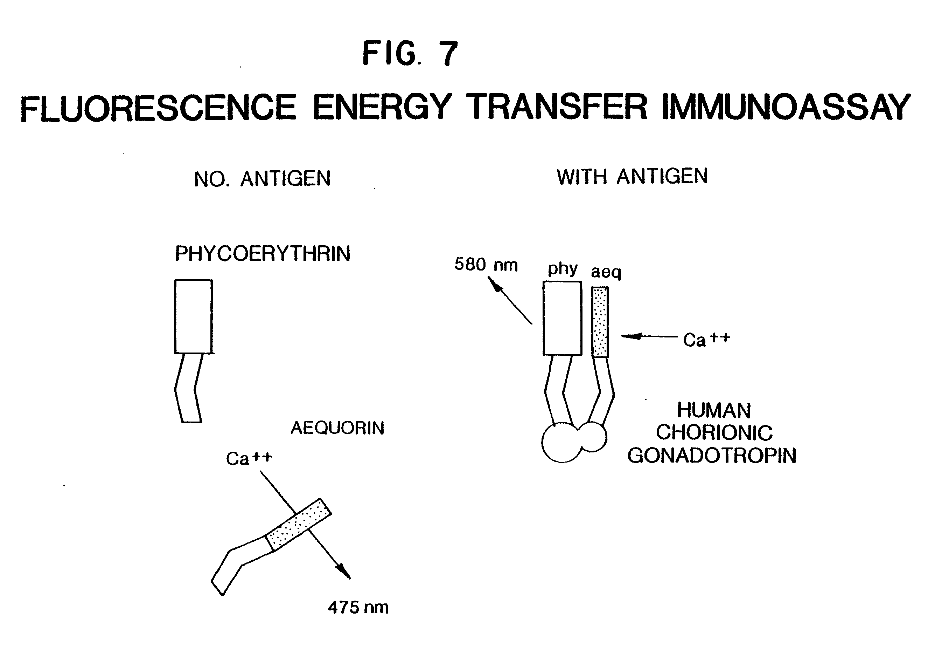

A closely related type of immunoassay, illustrated in Figure 7, involves luminescence energy transfer between a luminescent molecule and a fluorescent substance, as in Patel et al. (1983) Analytical Biochemistry 129, 162-169. Referring to Figure 7, the luminescent protein aequorin is used in conjunction with phycoerythrin. The presence of the antigen closely couples aequorin and phycoerythrin, greatly increasing energy transfer efficiency. The addition of Ca++ elicits the luminescence of aequorin at 475 nm, which excites phycoerythrin and causes it to emit fluorescence at 580 nm. Thus the 580 nm signal is only achieved by the wavelength modulation of aequorin luminescence by phycoerythrin, a process whose efficiency depends on the proximity of the proteins.

Other embodiments are within the following claims. For example, although IgG$_1$ antibodies are preferred, IgG$_2$ and IgA antibodies can also be used in the invention. Also, the entire half-molecule, rather than just the F(ab') portion, can be used. Yield can be improved slightly by using, as the dithiol complexing agent, instead of sodium arsenite, 0.25 mM phenylarsine oxide.

## Claims

1. A method of preparing a bispecific monoclonal antibody determinant comprising the steps of providing two different monoclonal antibody determinants, each said determinant comprising two identical L-H half-molecules linked by one or more disulfide bond,

subjecting said two different antibody determinants to conditions sufficient to break said disulfide bonds linking said L-H half-molecules, and under conditions which prevent the formation of disulfide bonds within the H-chains of said half-molecules, whereby each said determinant is split into a pair of identical half-molecules,

derivatizing one said pair of identical half-molecules with thiol activating agent, and

combining said half-molecules under conditions which permit said half-molecules to form said bispecific antibody determinants by the formation of one or more disulfide bonds.

2. The method of claim 1 wherein said two different antibody determinants are IgG or IgA.

3. The method of claim 2 wherein each different half-molecule consists of the F(ab') portion of an IgG antibody.

4. The method of claim 2 wherein one of said monoclonal IgG determinants comprises two identical L-H half-molecules linked by more than one disulfide bond.

5. The method of claim 4 wherein the formation of said disulfide bonds within said H chains is prevented by carrying out said splitting in the presence of a dithiol complexing agent.

6. The method of claim 5 wherein said dithiol complexing agent comprises an arsenite.

7. The method of claim 4 wherein the formation of said disulfide bonds within said H chains is prevented by carrying out said splitting under conditions under which the conformation of said H chains is modified to prevent said disulfide bond formation.

8. The method of claim 4 wherein the formation of said disulfide bonds within said H chains is prevented by, following said splitting, removing all but one reduced cysteine residues using a proteolytic enzyme.

9. The method of claim 1 wherein said thiol activating agent comprises an aromatic disulfide.

10. The method of claim 9 wherein said aromatic disulfide comprises 5,5'-dithiobis 5(2-nitrobenzoic acid).

11. The method of claim 9 wherein said aromatic disulfide comprises 2,2'-dipyridine disulfide.

12. The method of claim 9 wherein said aromatic disulfide comprises 4,4-dipyridine disulfide.

13. The method of claim 1 wherein said thiol activating agent comprises a mixture of sulfite and tetrathionate.

14. The method of claim 3 wherein, following the derivatization of both F(ab') half-molecules with a thiol activating agent, one of said half-molecules is converted from the available thiol-activated F(ab') to the free thiol.

15. The method of claim 1, further comprising the step of separating, from said bispecific antibody determinants, any unreacted said half-molecules.

**Revendications**

1. Méthode de préparation d'un déterminant bispécifique d'anticorps monoclonal comprenant les étapes de fourniture de deux déterminants d'anticorps monoclonaux différents, chacun des dits déterminants comprenant deux demi-molécules L-H identiques liées par un ou plusieurs ponts disulfures,

de soumission des dits deux déterminants d'anticorps différents à des conditions suffisantes pour la rupture des dits ponts disulfures liant les dites demi-molécules L-H, et dans des conditions qui empêchent la formation de ponts disulfures à l'intérieur des chaînes-H des dites demi-molécules, ce en quoi chacun des dits déterminants est clivé en une paire de demi-molécules identiques,

de dérivation d'une dite paire de demi-molécules identiques par un agent thiol activant, et

de combinaison des dites demi-molécules dans des conditions qui permettent aux dites demi-molécules de former les dits déterminants bispécifiques d'anticorps par la formation d'un ou plusieurs ponts disulfures.

2. Méthode de la revendication 1 dans laquelle les dits deux déterminants d'anticorps différents sont des IgG ou des IgA.

3. Méthode de la revendication 2 dans laquelle chaque demi-molécule différente consiste en la partie F(ab') d'un anticorps IgG.

4. Méthode de la revendication 2 dans laquelle l'un des dits déterminants d'IgG monoclonal comprend deux demi-molécules L-H identiques liées par plus d'un pont disulfure.

5. Méthode de la revendication 4 dans laquelle la formation des dits ponts disulfures à l'intérieur des dites chaînes H est empêchée par la réalisation du dit clivage en présence d'un agent complexant de dithiol.

6. Méthode de la revendication 5 dans laquelle le dit agent complexant de dithiol comprend un arsénite.

7. Méthode de la revendication 4 dans laquelle la formation des dits ponts disulfures à l'intérieur des dites chaînes H est empêchée par la réalisation du dit clivage dans des conditions où la conformation des dites chaînes H est modifiée afin d'empêcher la formation des dits ponts disulfures.

8. Méthode de la revendication 4 dans laquelle la formation des dits ponts disulfures à l'intérieur des dites chaînes H est empêchée par, suivant le dit clivage, l'élimination de tous les résidus cystéines réduits sauf un en utilisant une enzyme protéolytique.

9. Méthode de la revendication 1 dans laquelle le dit agent thiol-activant comprend un disulfure aromatique.

10. Méthode de la revendication 9 dans laquelle le dit disulfure aromatique comprend le 5,5'-dithiobis 5 (acide 2-nitrobenzoïque).

11. Méthode de la revendication 9 dans laquelle le dit disulfure aromatique comprend le 2,2'-dipyridine disulfure.

12. Méthode de la revendication 9 dans laquelle le dit disulfure aromatique comprend le 4,4-dipyridine disulfure.

13. Méthode de la revendication 1 dans laquelle le dit agent dithiol activant comprend un mélange de sulfite et de tétrathionate.

14. Méthode de la revendication 3 dans laquelle , à la suite de la dérivation des deux demi-molécules F(ab') par un agent thiol-activant, l'une des dites demi-molécules est convertie à partir du F(ab') thiol-activé disponible en thiol libre.

15. Méthode de la revendication 1, comprenant ultérieurement l'étape de sépation, à partir des dits déterminants bispécifiques d'anticorps , de toutes les dites demi-molécules n'ayant pas

réagi.

**Ansprüche**

1. Verfahren zum Erzeugen einer bispezifischen monoklonalen Antikörperdeterminante, das die Schritte aufweist: es werden zwei unterschiedliche monoklonale Antikörperdeterminante bereitgestellt, wobei jede Determinante zwei identische L-H Halbmoleküle enthält, die durch eine oder mehrere Disulfidbrücken miteinander verbunden sind,

es werden die beiden unterschiedlichen Antikörperdeterminanten Bedingungen ausgesetzt, die ausreichen, um die Disulfidbrückenverbindungen der L-H Halbmoleküle aufzubrechen und es liegen Bedingungen vor, die die Bildung von Disulfidbrücken zwischen den H-Ketten der Halbmoleküle verhindern, wobei jede Determinante in ein Paar identischer Halbmoleküle aufgespalten wird,

es wird mit den einen Paar der identischen Halbmoleküle mit Thiol aktivierendem Agens ein Derivat gebildet und

es werden die Halbmoleküle unter Bedingungen kombiniert, die es den Halbmolekülen ermöglichen, die bispezifischen Antikörperdeterminanten zu bilden, indem eine oder mehrere Disulfidbrücken gebildet werden.

2. Verfahren nach Anspruch 1, bei dem die beiden unterschiedlichen Antikörperdeterminanten IgG oder IgA sind.

3. Verfahren nach Anspruch 2, bei dem jedes der unterschiedlichen Halbmoleküle aus dem F-(ab')-Teil eines IgG Antikörpers besteht.

4. Verfahren nach Anspruch 2, bei dem eine der monoklonalen IgG Determinanten zwei identische L-H Moleküle enthält, die durch mehr als eine Disulfidbrücke miteinander verbunden sind.

5. Verfahren nach Anspruch 4, bei dem die Bildung der Disulfidbrücken zwischen den H-Ketten verhindert wird, indem die Aufspaltung in Anwesenheit eines Dithiolkomplex bildenden Agens ausgeführt wird.

6. Verfahren nach Anspruch 5, bei dem das Dithiolkomplex bildende Agens ein Arsenat aufweist.

7. Verfahren nach Anspruch 4, bei dem die Bildung der Disulfidbrücken zwischen den H-Ketten verhindert wird, indem das Aufteilen unter Bedingungen ausgeführt wird, unter denen die Konformation der H-Ketten modifiziert wird, um die Bildung der Disulfidbrücken zu verhindern.

8. Verfahren nach Anspruch 4, bei dem die Bildung der Disulfidbrücken zwischen den H-Ketten verhindert wird, indem nach dem Aufspalten durch Verwendung eines proteolytischen Enzyms bis auf einen alle reduzierten Cysteinreste entfernt werden.

9. Verfahren nach Anspruch 1, bei dem das Thiol aktivierende Agens ein aromatisches Disulfid aufweist.

10. Verfahren nach Anspruch 9, bei dem das aromatische Disulfid 5,f'-Dithiobis 5(2-Nitrobenzoesäure) enthält.

11. Verfahren nach Anspruch 9, bei dem das aromatische Disulfid 2,2'-Dipyridindisulfid ist.

12. Verfahren nach Anspruch 9, bei dem das aromatische Disulfid 4,4-Dipyridindisulfid ist.

13. Verfahren nach Anspruch 1, bei dem das Thiol aktivierende Agens eine Mischung aus Sulfid und Tetrathionat enthält.

14. Verfahren nach Anspruch 3, bei dem nach dem Bilden des Derivats beider F (ab')-Halbmoleküle mit einem Thiol aktivierenden Agens eines der Halbmoleküle aus dem verfügbaren Thiol aktivierten F(ab') in freies Thiol umgewandelt ist.

15. Verfahren nach Anspruch 1, das ferner den Schritt aufweist: es werden von den bispezifischen Antikörperdeterminanten alle Halbmoleküle abgetrennt, die nicht reagiert haben.

# BIOLUMINESCENCE ASSAY FOR LACTOSE

**FIG. 1**

EP 0 179 872 B1

# CHANNELING IMMUNOASSAY

FIG. 2

# FIG. 3

## FLUORESCENCE ENERGY TRANSFER IMMUNOASSAY

NO. ANTIGEN

WITH ANTIGEN

PHYCOERYTHRIN

500nm → 580nm

ALLOPHYCOCYANINE

500nm

500nm → 660nm

HUMAN CHORIONIC GONADOTROPIN

| DISTANCE BETWEEN CHROMOPHORES: | ENERGY TRANSFER EFFICIENCY: |
|---|---|
| 110 Å | 10% |
| 68 Å | 50% |

EP 0 179 872 B1

# FIG. 4

## THE IMMUNOGLOBULIN MOLECULE

RABBIT IgG$_1$

MOUSE IgG$_1$

# DIMER FORMATION (INTERCHAIN DISULFIDE)

# COMPETING SIDE REACTION (INTRACHAIN DISULFIDE)

CONFORMATIONAL EQUILIBRIUM

## FIG. 5

EP 0 179 872 B1

# FIG. 6

## METHOD FOR SELECTIVE RECOMBINATION OF MONOCLONAL Fab′ FRAGMENT

1. PREPARATION OF Fab′ THIONITROBENZOATE (TNB) DERIVATIVES

2. PREPARATION OF Fab′ THIOL ARSENITE DERIVATIVES

3. PREPARATION OF PURE BISPECIFIC ANTIBODY

EP 0 179 872 B1

# FIG. 7

## FLUORESCENCE ENERGY TRANSFER IMMUNOASSAY

NO. ANTIGEN

WITH ANTIGEN

PHYCOERYTHRIN

580 nm    phy    aeq

Ca$^{++}$

AEQUORIN

Ca$^{++}$

HUMAN
CHORIONIC
GONADOTROPIN

475 nm

EP 0 179 872 B1